# EUROPÄISCHE PATENTSCHRIFT

(11) **EP 2 099 512 B1**
(45) Veröffentlichungstag und Bekanntmachung des Hinweises auf die Patenterteilung: **21.04.2010**
(21) Anmeldenummer: 07846333.8
(22) Anmeldetag: 14.11.2007
(51) Int. Cl.: A61M 1/00

(54) **APPLIKATOR FÜR EINE WASSERSTRAHLTRENNEINRICHTUNG, INSBESONDERE ZUR BEHANDLUNG VON WUNDEN UND GESCHWÜREN**
APPLICATOR FOR A WATER JET SEPARATING DEVICE, PARTICULARLY FOR THE TREATMENT OF WOUNDS AND ABSCESSES
APPLICATEUR POUR UN DISPOSITIF SEPARATEUR A JET D'EAU, NOTAMMENT POUR LE TRAITEMENT DE BLESSURES ET D'ULCERES

(30) Priorität: 16.12.2006 DE 202006018986 U
(43) Veröffentlichungstag der Anmeldung: 16.09.2009
(73) Patentinhaber: Human Med AG, 19061 Schwerin (DE)
(72) Erfinder: KENSY, Arnd, 14552 Michendorf (DE); WINKLER, Konrad-Wenzel, 19417 Warin (DE); NIKLAS, Frank, 19386 Lübz (DE); PEULECKE, Thomas, 19059 Schwerin (DE); RUNOW, Andreas, 19055 Schwerin (DE); BEHNERT, Fred, 19073 Dümmer (DE); SCHLEE, Gernot, 19057 Schwerin (DE)
(74) Vertreter: Gulde Hengelhaupt Ziebig & Schneider
(86) Internationale Anmeldenummer: PCT/DE2007/002057
(87) Internationale Veröffentlichungsnummer: WO 2008/074284

(56) Entgegenhaltungen:
- DE-A1- 4 018 736
- DE-T2- 69 927 153
- US-A- 5 037 431
- US-A- 5 941 859
- US-A1- 2004 243 157

## Beschreibung

Die Erfindung bezieht sich auf einen Applikator nach dem Oberbegriff des Anspruchs 1. Derartige Wasserstrahltrenneinrichtungen werden in der Human- und Veterinärmedizin eingesetzt.

Wunden oder Geschwüre sind Beschädigungen der Haut, die immer dann am besten abheilen, wenn eine ausreichende Eigenblutung zur Selbstreinigung besteht, wenn die im Geschwür sich bestens entwickelnden Bakterien vollständig abgetötet oder entfernt werden und wenn die obere Epidermis des Gewebes zum besseren Zusammenwachsen scharfkantig ausgebildet ist. Bei kleineren Wunden muss daher lediglich gereinigt und desinfiziert werden, um diese Bedingungen zu erfüllen.

Größere Wunden und Geschwüren haben in der Regel unscharfe Wundränder und Geschwüre weisen einen großen Anteil an nekrotischem Gewebe auf. Dabei ist das nekrotische Gewebe meistens großflächig und häufig auch tiefgehend ausgebildet, was den Heilungsprozess verhindert und die Gefahr einer Infektion stark erhöht. Ein besonders gefährliches und behandlungsintensives Geschwür ist das Bein- oder Unterschenkelgeschwür (Ulkus cruris).
Geschwüre werden daher in der Regel mechanisch bearbeitet, in dem das nekrotische Gewebe mit Hilfe einer Kürette oder eines Skalpells ausgeschabt bzw. ausgeschnitten wird, wobei zur Verbesserung der Eigenblutung bis in das gesunde Gewebe eingedrungen wird.

Dieses Verfahren ist aber für den ausführenden Arzt nicht sehr angenehm und für den Patienten nicht ausreichend schmerzarm.
Es besteht daher in der Fachwelt für diesen Einsatzfall die Forderung nach einer in der Medizintechnik bereits weitgehend eingeführten Wasserstrahltrenntechnik.
Zu dieser Wasserstrahltrenntechnik gehört grundsätzlich eine Druckerzeugungseinrichtung mit einer Austrittsdüse für einen trennenden Flüssigkeitsstrahl, eine Vakuumerzeugungseinrichtung mit einer oder mehreren Saugöffnungen für einen Saugstrom und ein Applikator, der auf den speziellen Einsatzfall angepasst ist und der dazu die Austrittsdüse für den trennenden Flüssigkeitsstrahl und die Saugöffnungen für den Saugstrom in besonderer Weise ausbildet und zueinander anordnet. Diese Wasserstrahltrenntechnik wird erfolgreich invasiv und immer dort angewendet, wo Körperhöhlen vorhanden sind, in denen sich die Trennflüssigkeit örtlich ansammeln kann, damit sie danach ungehindert zusammen mit den abgetrennten Gewebeteilen wieder abgesaugt werden kann. Diese Bedingung gibt es bei der Wundbehandlung im Allgemeinen und insbesondere vom Ulkus cruris nicht. Der Einsatz der herkömmlichen Wasserstrahltrenntechnik ist daher in diesen Fällen ungeeignet, weil sich die Trennflüssigkeit und damit die vorhandenen krankheitserregenden Mikroorganismen aus dem Geschwür unkontrolliert in das Umfeld der Operation verteilen könnten. Das gefährdet den Patienten und das Personal.

US 5 037 431 offenbart ein Gerät zur Wandspülung, das die Merkmale des Oberbegriffs von Anspruch 1 aufweist.

Aus der US 5 941 859 ist ein Wundenspülgerät mit einer Flüssigkeitsrückführung bekannt, das ein glockenförmiges Schutzschild aufweist. Innerhalb dieses Schutzschildes befindet sich eine Austrittsdüse für ein flüssiges Medikament und ein Saugstutzen für das ausgetretene flüssige Medikament. Der Schutzschild bedeckt die Wunde während der Behandlung und schützt somit den Operateur vor der mit Bakterien angereicherten Flüssigkeit. Dieser Schutz ist aber nur gering, weil keine ausreichende Abdeckung des Arbeitsbereiches vorhanden ist. Außerdem ist das Wundspülgerät nur zum Spülen und Reinigen einer Wunde vorgesehen, ein chirurgischer Eingriff zur Behandlung der Wunde ist nicht möglich.

In einer Veröffentlichung von Guthke, Jage, Wendekamm und Kühne "Wundreinigung bei Ulcus cruris - eine Indikation für den Hochdruckwasserdissektor"; Zbl Haut 1994: 164; 181-2 wurde ein gegen das Umfeld abschirmendes Behandlungszelt mit einer Abzugsschleuse der Firma Medaxis vorgestellt, die den gesamten Arbeitsplatz für die Operation einhaust und so eine unkontrollierte Ausbreitung von Mikroorganismen vermeidet. Diese Abzugsschleuse entspricht nicht den heutigen Anforderungen, weil sie die Handlungsfähigkeit des Operateurs stark einschränkt und weil sie eine außergewöhnlich starke Saugleistung benötigt, um den aus Luft, Wasser und Gewebeteilen bestehenden Inhalt der großvolumigen Abzugsschleuse abzusaugen. Dementsprechend groß und stark müssen die Antriebseinrichtungen ausgelegt sein und so ist es nicht möglich, diese Wasserstrahltrenneinrichtung für die ambulante Wundbehandlung einzusetzen.

Es sind nun eine Reihe von Applikatoren bekannt, bei denen die Austrittsdüse der Druckleitung und die Eintrittsöffnung der Saugleitung so einander zugewandt angeordnet sind, dass der austretende Wasserstrahl zusammen mit den abgetrennten Gewebeteilen direkt in die Saugöffnung eintritt.
So beschreibt beispielsweise die EP0 997 105 B1 einen Applikator, bei dem die Austrittsdüse als ein Düsenring mit einen umlaufenden und radial ausgerichteten Düsenschlitz ausgebildet ist. Dieser Schlitz ist dabei so ausgerichtet und die Drücke des Flüssigkeitsstrahles und des Saugstromes sind so aufeinander abgestimmt, dass sich eine in das Saugrohr eintauchende Flüssigkeitsmembran ausbildet, die das Gewebe trennt.
Die DE 600 23 136 T2 (EP 1 182 974 B1) beschreibt einen Applikator, bei dem die Austrittsdüse der Wasserstrahls und die Eintrittsöffnung der Saugleitung auf einen bestimmten Abstand zueinander gegenübergestellt sind. Dabei ist der Abstand so gewählt, dass nach dem Prinzip von Venturi eine zusätzliche Saugkraft erzeugt wird, die den Abtransport des abgetrennten Gewebes verbessert. In besonderen Fällen kann auf eine gesonderte Saugeinrichtung verzichtet werden.

In der DE 40 18 736 A1 ist dagegen ein Applikator beschrieben, der am distalen Ende des Saugrohres einen Düsenring mit zwei Austrittsdüsen für die Trennflüssigkeit besitzt, die radial und in das Innere des Saugrohres ausgerichtet sind.
Alle diese Applikatoren vermeiden zwar wegen der unmittelbaren Nähe der Austrittsdüse und der Saugöffnung ein unkontrolliertes Streuen der austretenden Trennflüssigkeit, haben aber gemeinsam den Nachteil, dass sie nur ein sehr geringes Arbeitsfeld ausbilden. Der Abstand zwischen der Austrittsdüse und der Saugöffnung ist aber funktionsbedingt gering und so ist eine Vergrößerung dieses Abstandes nur begrenzt möglich, weil die Größe der Saugöffnung und die Stärke des Saugstromes ein einwandfreies Aufnehmen des gesamten Flüssigkeitsstromes dann nicht mehr gewährleisten können. Diese Applikatoren sind ausschließlich für den invasiven Einsatz vorgesehen und daher für ein Debridement von offenen und großflächigen Wunden und Geschwüren ungeeignet.

Der Erfindung liegt daher die Aufgabe zu Grunde, einen gattungsgemäßen Applikator zu entwickeln, der ein größtmögliches Arbeitsfeld schafft und dabei das Umfeld abschirmt und gleichzeitig für den mobilen Einsatz geeignet ist.
Diese Aufgabe wird durch die kennzeichnenden Merkmale des Anspruches 1 gelöst. Der neue Applikator beseitigt die genannten Merkmale des Standes der Technik.
Dabei kommen alle die bekannten Vorteile der Wasserstrahltrennung zum Tragen, die insbesondere darin bestehen, dass in schonender und für den Patienten verträglicher Weise bis tief in das gesunde Gewebe eingeschnitten werden kann. Dazu gehören beispielsweise die Verwendung eines dynamischen und gepulsten Wasserstrahls, die Verwendung eines abrasiven Trennmittels, die Verwendung eines antibiotischen, antiseptischen Mediums oder der Einsatz eines temperaturgeregelten Trennmittels. Dabei ist es besonders zweckmäßig, wenn in der Abdeckhaube über die Saugleitung ein vorzugsweise pulsierendes, das Gewebe stimulierendes Vakuum erzeugt wird. Das alles fördert den Heilungsprozess. Der spezielle Vorteil des neuen Applikators liegt nun darin, dass der Eingriff in einer Vakuum verschlossenen Arbeitskammer erfolgt. Das saugt die Gewebeteile in die Arbeitskammer und präpariert sie in vorteilhafter Weise für den Trennvorgang. Das Vakuum sorgt aber auch dafür, dass die Durchblutung der Wunde oder des Geschwürs verstärkt wird, was sich vorteilhaft auf die Reinigung der Wunde oder des Geschwürs auswirkt. Ein besonderer Vorteil liegt aber auch darin, dass das Ausbreiten von krankheitserregenden Mikroorganismen zum Schutz des Operateurs und des Umfeldes sicher vermieden wird. Dieser Schutz tritt durch das vakuumbedingte Abdichten der Arbeitskammer während des chirurgischen Eingriffs ein. Der Schutz ergibt sich aber auch dadurch, dass die Trennflüssigkeit vom Vakuum gesteuert nur dann austritt, wenn die Abdichtung der Arbeitskammer gewährleistet ist. Durch ein spezielles Dichtelement mit einer neuartigen Fangklappe ist eine zusätzliche Abschirmung während der Abschaltphase vorgesehen.
Der neue Applikator schafft auch ein vergrößertes Arbeitsfeld, weil die Abdeckhaube unabhängig von der Anordnung der Austrittsdüsen und der Saugöffnungen dimensioniert, gestaltet und angeordnet werden kann. Dadurch können größere Wund- oder Geschwürflächen bearbeitetet werden. Sollte dennoch ein Umsetzen erforderlich werden, ist das vom Operateur durch die Steuerung des Vakuums über die Bypassöffnung im Saugstrom leicht zu realisieren.
Zweckdienliche Ausgestaltungen ergeben sich aus den Unteransprüchen 2 bis 16.

Die Erfindung soll anhand mehrerer Ausführungsbeispiele näher erläutert werden.
Dazu zeigen:
- Fig. 1:: einen Schnitt durch einen Applikator in einer ersten Ausführungsform,
- Fig. 2:: eine Seitenansicht des Applikators nach der Fig. 1,
- Fig. 3:: eine Draufsicht des Applikators nach der Fig. 1,
- Fig. 4:: einen Schnitt durch einen Applikator in einer zweiten Ausführungsform,
- Fig. 5:: eine Seitenansicht des Applikators nach der Fig. 4,
- Fig. 6:: eine Draufsicht des Applikators nach der Fig. 4,
- Fig. 7:: eine Draufsicht des Applikators nach der Fig. 4 mit einer abgewandelten Düsenanordnung,
- Fig. 8:: einen Längsschnitt durch einen Applikator in einer dritten Ausführungs-form,
- Fig. 9:: einen Querschnitt durch den Applikator nach der Fig. 8,
- Fig. 10:: einen Längsschnitt durch einen abgewandelten Applikator der dritten Ausführungsform nach der Fig. 8,
- Fig. 11:: einen anderen Längsschnitt des abgewandelten Applikators nach der Fig. 10,
- Fig. 12:: eine Seitenansicht eines Applikators in einer vierten Ausführungsform und
- Fig. 13:: ein gegenüber der Fig. 12 abgewandelter Applikator.

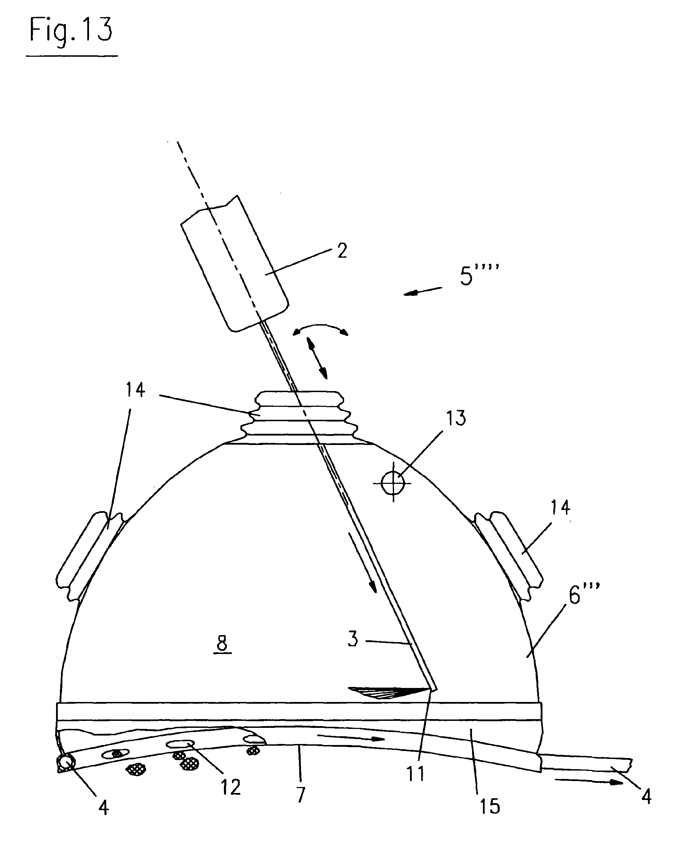

Der Applikator 1 gehört zu einer chirurgische Wasserstrahltrenneinrichtung, die aus einer Flüssigkeitstrenneinrichtung zum Trennen einer biologischen Struktur und einer entsprechenden Absaugeinrichtung besteht. Diese Wasserstrahltrenneinrichtung ist in der Medizintechnik inzwischen allgemein bekannt und braucht daher nicht näher gezeigt und beschrieben zu werden. Die Wasserstrahltrenneinrichtung besteht demnach aus einem Vorratsbehälter für eine sterile Flüssigkeit, einer Druckpumpe und einer Einspritzleitung und die Absaugeinrichtung besitzt einen Auffangbehälter für die Flüssigkeit und die abgetrennten Gewebeteile, eine Saugpumpe und eine Absaugleitung. Die Einspritzleitung der Wasserstrahltrenneinrichtung und die Absaugleitung der Absaugeinrichtung münden gemeinsam in den Applikator 1.
Der Applikator 1 aller Ausführungsformen außer Fig. 13 besteht aus einem Handstück 2, das die Druckleitung 3 für den austretenden Wassertrennstrahl und die Saugleitung 4 für die aufgenommene Flüssigkeit und die abgetrennten Gewebeteile zusammenhält und sie zu einer, am distalen Ende des Applikators 1 befindlichen Arbeitssonde 5 führt. Dabei sind die Druckleitung 3 und die Saugleitung 4 getrennt voneinander, unmittelbar benachbart und achsparallel zueinander angeordnet. In der Saugleitung 4 befindet sich im Griffbereich des Handstückes 2 eine Bypassöffnung 13.

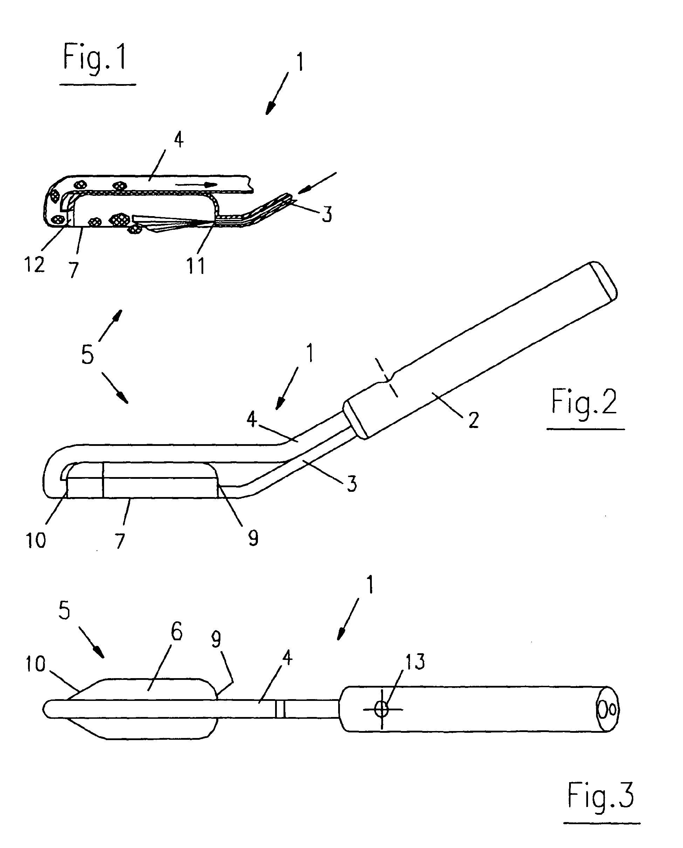

Nach den Fig. 1 - 3 besitzt die Arbeitssonde 5 in der ersten Ausführungsform eine flache, nach unten offene und nach oben und zu den Seiten geschlossene Abdeckhaube 6, die auf ihrer nach unten offenen Seite eine umlaufende Dichtkante 7 besitzt. Die Dichtkante 7 ist dabei weitestgehend an die Körperformen des Patienten anpassbar und vorzugsweise mit einem besonderen Dichtmaterial ausgerüstet, das einen luftdichten Abschluss zur Körperoberfläche ermöglicht. Damit bildet sich eine hermetisch abgeschlossene Arbeitskammer 8 aus. Die Abdeckhaube 6 besteht aus einem transparenten Material, um eine Sicht von außen in die Arbeitskammer 8 und damit auf das Arbeitsfeld zu ermöglichen. Diese Abdeckhaube 6 ist vorzugsweise länglich gestreckt mit einer zum Handstück 2 nahen Seitenfläche 9 und einer zum Handstück 2 fernen Seitenfläche 10 ausgeformt. Die Druckleitung 3 für den Wasserstrahl mündet seitlich in der zum Handstück 2 nahen Seitenfläche 9 und bildet dort eine Austrittsdüse 11 aus. Diese Austrittsdüse 11 ist vorzugsweise eine Flachdüse. Die Saugleitung 4 ist oberhalb der Abdeckhaube 6 zur zum Handstück 2 fernen Seitenfläche 10 geführt, in der sie an der tiefsten Stelle der Abdeckhaube 6 einmündet und in diesem Bereich eine Saugöffnung 12 besitzt. Damit liegt die Saugöffnung 12 der Austrittsdüse 11 in gleicher Ebene gegenüber und damit im Wirkungsbereich der Austrittsdüse 11. Zur besseren Aufnahme des Wassertrennstrahls und der abgetrennten Gewebeteile ist die Abdeckhaube 6 im Bereich der Saugöffnung 12 trichterförmig ausgebildet. Diese Abdeckhaube 6 schirmt das Arbeitsfeld des Operateurs gegenüber dem Umfeld hermetisch ab.

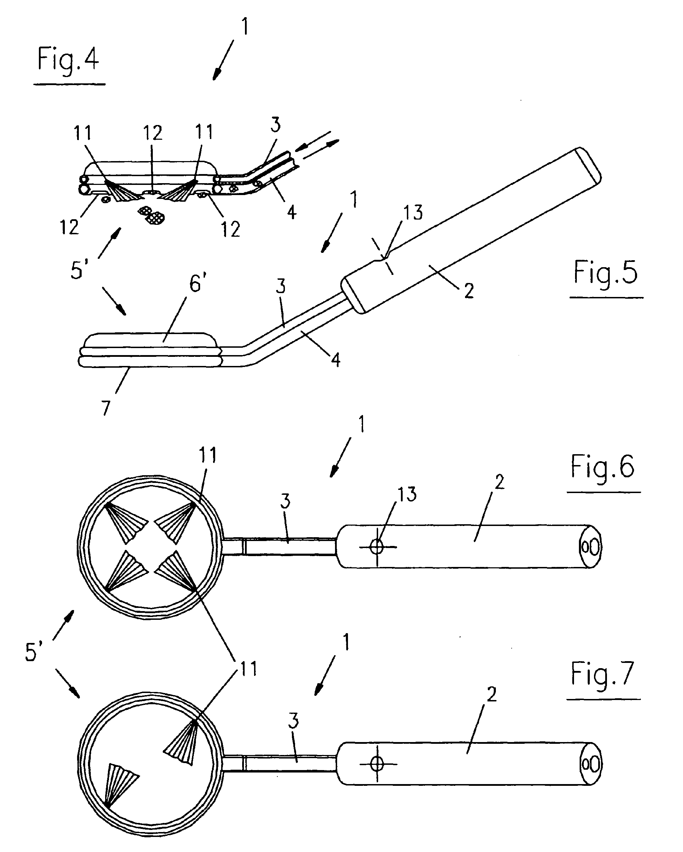

Nach den Fig. 4-7 besitzt die Arbeitssonde 5' in der zweiten Ausführungsform in gleicher Weise ein Handstück 2 mit einer Druckleitung 3, einer Saugleitung 4 und einer Abdeckhaube 6' mit einer Dichtkante 7, wobei die Druckleitung 3 und die Saugleitung 4 wiederum der Abdeckhaube 6' seitlich zugeführt sind. Dabei ist die Abdeckhaube 6' im Querschnitt rund ausgeführt und die Druckleitung 3 und die Saugleitung 4 am Umfang der Abdeckhaube 6' herumgeführt. Der umlaufende Teil der Saugleitung 4 befindet sich in der untersten Ebene und die Druckleitung 3 in der darüber liegenden Ebene der Abdeckhaube 6'. Dazu befinden sich im umlaufenden Teil der Saugleitung 4 vorzugsweise mehrere gleichmäßig verteilt angeordnete Saugöffnungen 12. Die darüber liegende Druckleitung 3 besitzt nach der Fig. 6 vier gleichmäßig versetzt angeordnete Austrittsdüsen 11, sodass immer zwei der Austrittsdüsen 11 sich gegenüberliegen. Dabei sind die Austrittsdüsen 11 auf die darunter liegende Ebene gerichtet, in der sich die Saugöffnungen 12 befinden. Die vier oder auch mehr, möglichst in gerader Anzahl vorgesehenen Austrittsöffnungen 12 bilden somit in der Mitte der Abdeckhaube 6' einen Fokus.
Nach der Fig. 7 sind lediglich zwei der Austrittsöffnungen 11 vorgesehen, die sich gegenüberliegen und in einer Ebene befinden, aber seitlich aneinander vorbei gerichtet sind. Dabei sind diese Verhältnisse so gewählt, dass ein von den Wassertrennstrahlen der beiden Austrittsdüsen 11 angegriffenes Gewebeteil in Rotation versetzt wird. Das unterstützt die Trennung.

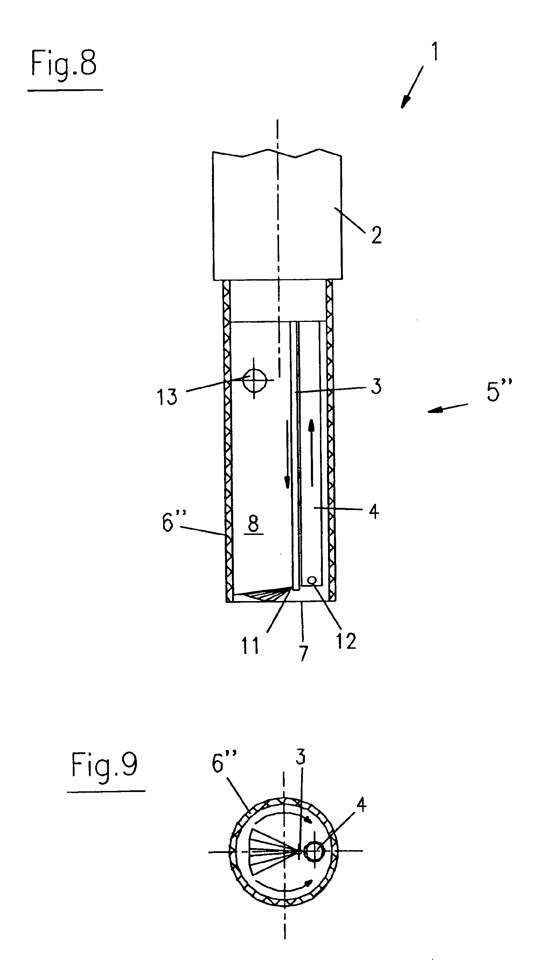

Nach den Fig. 8 und 9 besitzt die Arbeitssonde 5" eine zylindrische Abdeckhaube 6", die die Druckleitung 3 und die Saugleitung 4 im distalen Bereich des Applikators 1 in der Länge aufnimmt und umhüllt und fest mit dem Handgriff 2 verbunden ist. Am distalen Ende ist die Abdeckhaube 6" offen ausgeführt, sodass sich eine kreisrunde Dichtkante 7 ausbildet. Dabei sind die Druckleitung 3 und die Saugleitung 4 wieder achsparallel zueinander und zur zylindrischen Abdeckhaube 6" angeordnet. Zusammen sind die Druckleitung 3 und die Saugleitung 4 in besonderer Weise außermittig zur zylindrischen Abdeckhaube 6" in der Art angeordnet, dass sich die Saugleitung 4 achsfern und in unmittelbarer Nähe zur Innenwand der zylindrischen Abdeckhaube 6" und die Druckleitung 3 auf der achsnahen Seite der Saugleitung 4 befinden.
Die Saugleitung 4 besitzt am distalen Ende eine stirnseitige Saugöffnung 12 und wahlweise weitere Saugöffnungen 12, die am Umfang verteilt angeordnet sind. Dementsprechend endet die Saugleitung 4 in der Länge mit einem vorbestimmten Abstand oberhalb der Dichtkante 7 der Abdeckhaube 6", um die stirnseitige Saugöffnung 12 offen zu halten. Die Druckleitung 3 besitzt eine Austrittsdüse 11, die am äußersten distalen Ende und radial angeordnet ist und in Richtung weg von der Saugleitung 4 zeigt.

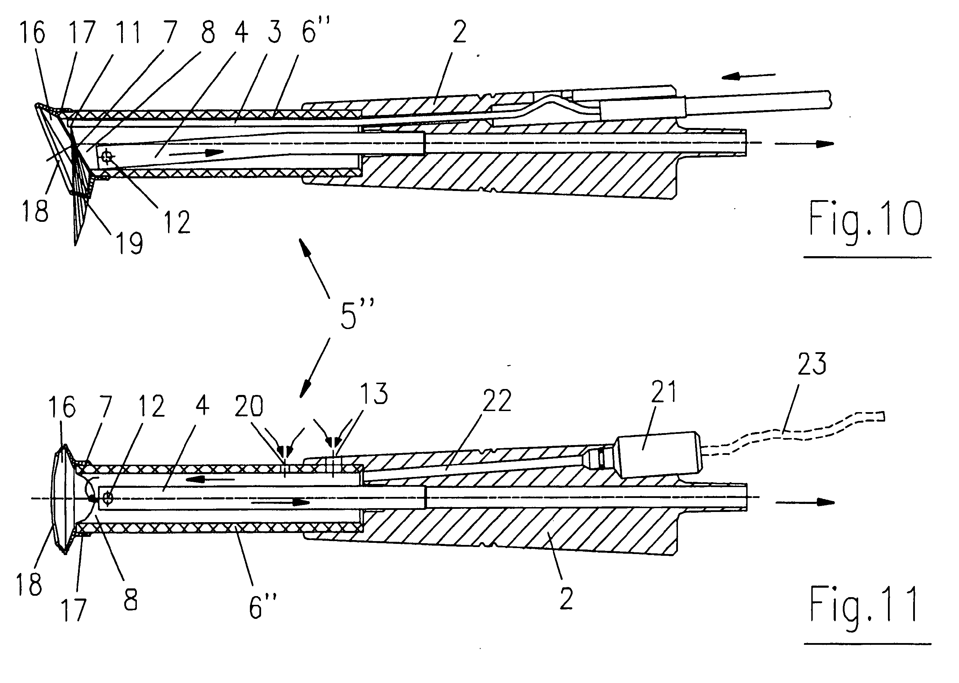

Die abgewandelte Arbeitssonde 5" in der dritten Ausführungsform nach den Fig. 10 und 11 besitzt wiederum eine zylindrische Abdeckhaube 6", die aus einem transparenten Material besteht und die die Druckleitung 3 und die Saugleitung 4 im distalen Bereich des Applikators 1 in der Länge aufnimmt und umhüllt und die fest mit dem Handgriff 2 verbunden ist. Dabei sind die Druckleitung 3 und die Saugleitung 4 mit einem radialen Abstand zueinander und zur zylindrischen Abdeckhaube 6" in der Art angeordnet, dass die Enden der Druckleitung 3 und der Saugleitung 4 im distalen Bereich mit einem größten radialen Abstand zueinander angeordnet sind. Die Druckleitung 3 ist stirnseitig verschlossen, wobei die Austrittsdüse 11 radial und am äußersten Ende der Druckleitung 3 angeordnet ist. Die Austrittsdüse 11 ist vorzugsweise eine Flachdüse. Dabei zeigt die Austrittsdüse 11 in Richtung des Endes der Saugleitung 4. Dieses Ende der Saugleitung 4 besitzt eine stirnseitige und wahlweise weitere radiale Saugöffnungen 12.
Die Abdeckhaube 6" ist am distalen Ende wiederum offen ausgeführt. In der vorliegenden Ausführung verläuft das Ende der Abdeckhaube 6" abgeschrägt, sodass sich eine ovale Dichtkante 7 ausbildet. Auf diese Dichtkante 7 ist ein spezielles Dichtelement 16 aufgesetzt, das aus einem flexiblen Material besteht und somit an verschiedene Körperformen des Patienten anpassbar ist. Dieses Dichtelement 16 besitzt einen proximalen Haltering 17, der die Verbindung zur zylindrischen Abdeckhaube 6" gewährleistet, und eine distale Dichtlippe 18, die auf der Körperoberfläche des Patienten aufliegt. In besonderer Weise ist die distale Dichtlippe 18 im Bereich der Saugöffnungen 12 der Saugleitung 4 mit einer in axialer Richtung beweglichen Fangklappe 19 ausgestattet. Dabei ist die Fangklappe 19 zwischen einer ausgeklappten Stellung in der Ruheposition, so wie es die Fig. 10 zeigt, und einer nicht dargestellten eingeklappten Stellung in der aktiven Einsatzposition beweglich.
Die Abdeckhaube 6" besitzt im Bereich des Handstückes 2 ebenfalls eine erste Bypassöffnung 13, die von der Hand des Operateurs offen oder geschlossen gehalten wird und so bemessen ist, dass im offenen Zustand der Saugstrom über die Bypassöffnung 13 geleitet und damit ein Aufbau von Saugkräften am distalen Ende verhindert wird. In der Nähe dieser ersten Bypassöffnung 13 befindet sich eine zweite Bypassöffnung 20, die in Abstimmung mit der Leistung der Vakuumpumpe so bemessen ist, dass sich bei einer vollständigen Abdichtung am Dichtelement 16 und einer geschlossenen Bypassöffnung 13 ein definierter Saugstrom mit definierten Saugkräften einstellt.
Im Handstück 2 des Applikators 1 befindet sich weiterhin ein Vakuumsensor 21, der einerseits über eine Druckfühlerleitung 22 mit der Arbeitskammer 8 und andererseits über eine Signalleitung 23 mit der Flüssigkeitsförderpumpe verbunden ist. Dabei ist der Vakuumsensor 21 so ausgeführt und so mit der Flüssigkeitsförderpumpe gekoppelt, dass die Flüssigkeitsförderpumpe nur bei einem vorbestimmten Vakuumdruckbereich förderfähig ist. Eine Flüssigkeitsförderung und damit ein Austritt von Flüssigkeit aus der Austrittsdüse 11 sind außerhalb dieser Vakuumdruckdifferenz nicht möglich.

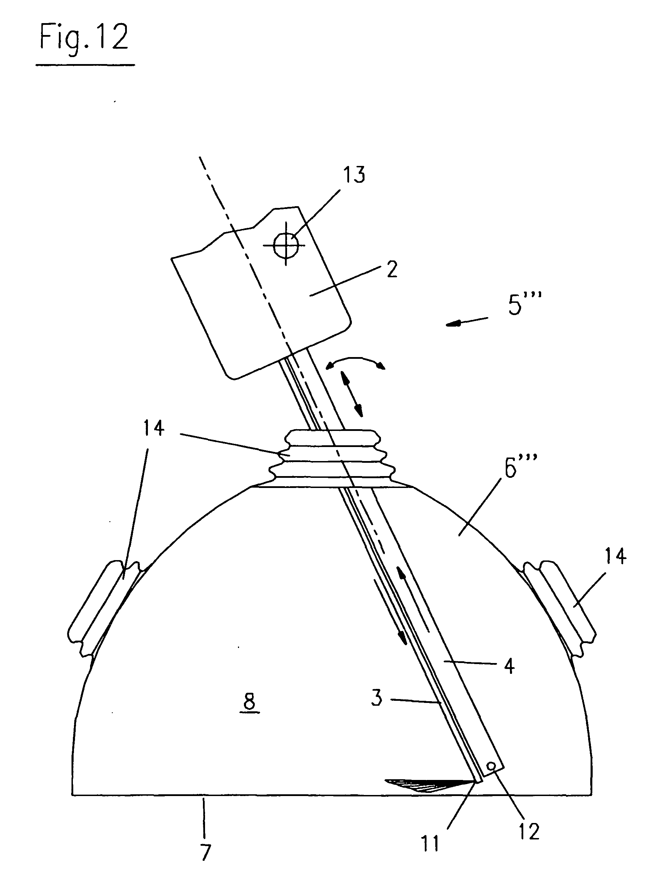

Nach der Fig. 12 besitzt die Arbeitssonde 5" eine als Glocke ausgebildete Abdeckhaube 6"', die im unteren Bereich wiederum offen und mit einer kreisrunden Dichtkante 7 ausgestattet ist. In der Glockenkuppel der Abdeckhaube 6'" befindet sich eine Öffnung, in der die wiederum achsparallel nebeneinander liegenden Druckleitung 3 und Saugleitung 4 hindurchgeführt sind. In dieser Öffnung der Glockenkupplung ist ein flexibles Halte- und Dichtelement 14 eingesetzt, welches die Druckleitung 3 und die Saugleitung 4 hält und den Zwischenraum zwischen der Abdeckhaube 6"' und der Druckleitung 3 und der Saugleitung 4 nach außen abdichtet. Dabei ist Halte- und Dichtelement 14 fest mit der Abdeckhaube 6'" verbunden und gegenüber der Druckleitung 3 und der Saugleitung 4 gleitend ausgelegt. Damit ist die Druckleitung 3 und die Saugleitung 4 sowohl in der Höhe als auch in der Winkelstellung verstellbar, sodass die distalen Enden der Druckleitung 3 und der Saugleitung 4 zusammen jeden Punkt der Arbeitsfläche innerhalb der Abdeckhaube 6'" erreichen können. Neben dem flexiblen Halte- und Dichtelement 14 in der Glockenkupplung oder alternativ dazu, können weitere jeweils mit einem Halte- und Dichtelement 14 verschlossene Öffnungen zum wahlweisen Hindurchführen der Druckleitung 3 und der Saugleitung 4 vorgesehen sein. Damit kann die Zugänglichkeit zum Arbeitsfeld des Operateurs verbessert werden.

Die distalen Enden der Druckleitung 3 und der Saugleitung 4 sind wiederum in bekannter Weise mit einer radialen und von der Saugleitung 4 weg gerichteten Austrittsdüse 11 und einer stirnseitigen und wahlweise weiterer radialer Saugöffnungen 12 ausgestattet.

Gemäß der Fig. 13 besitzt die Abdeckhaube 6'" im Bereich seiner Dichtkante 7 ein umlaufendes Dichtelement 15, das auswechselbar an der Abdeckhaube 6'" befestigt ist und mit seiner Dichtkante 7 an bestimmte Körperteile des Patienten angepasst ist. Dieses Dichtelement 15 kann gegen andere Dichtelemente 15 mit einer anderen Kontur der Dichtkante 7 ausgewechselt werden. Das Dichtelement 15 kann auch aus einem flexiblen Material bestehen und damit selbstanpassend ausgeführt sein. Im Dichtelement 15 ist die ebenfalls umlaufend ausgeführte Absaugleitung 4 integriert und direkt abgeleitet, wobei die Absaugleitung 4 in seinem umlaufenden Bereich mehrere Saugöffnungen 12 aufweist. Dementsprechend ist durch das Halte- und Dichtelement 14 lediglich die Druckleitung 3 hindurch geführt, wodurch während der Operation auch nur diese Druckleitung 3 gehandhabt wird.

Während des Betriebes der Wasserstrahltrenneinrichtung stellt sich in der Druckleitung 3 ein vorbestimmter Wasserdruck ein, der im Zusammenwirken mit der Austrittsdüse 11 einen entsprechenden vorzugsweise flachen Wassertrennstrahl ausbildet. Die Saugleitung 4 ist zunächst drucklos, weil die Bypassöffnung 13 geöffnet ist und so einen Nebensaugstrom erzeugt. Mit dem Verschluss dieser Bypassöffnung 13 durch den Operateur baut sich in der Arbeitskammer 8 der Abdeckhaube 6, 6', 6", 6'" ein Unterdruck auf, der das im Bereich der Arbeitssonde 5, 5', 5", 5"', 5"" befindliche Gewebe anhebt und es gegen die Dichtkante 7 der Abdeckhaube 6, 6', 6", 6"' drückt. Damit ist das Arbeitsfeld gegenüber dem Umfeld hermetisch abgeschirmt, sodass kein Wasser und damit keine Bakterien aus dem Arbeitsfeld austreten können. Gleichzeitig wird das abzutrennende Gewebe durch die Kraft des Saugstromes innerhalb der Abdeckhaube 6 angehoben und in eine starke Straffung und in eine günstige Position für den Trennvorgang gebracht. Der Wasserstrahl verrichtet dann Arbeit, in dem er in intelligenter Weise zwischen die Gewebezellen dringt, sie aufweitet und weiter spannt und sie voneinander trennt. Gewebezellen werden dabei kaum beschädigt. Der Saugstrom erfasst die abgetrennten Gewebeteile zusammen mit dem sich ansammelnden Wasser und befördert sie durch die Saugöffnungen 12 zum Aufnahmebehälter.
Bei der ersten Ausführungsform gemäß der Fig. 1 - 3 sind der Trennstrahl und der Saugstrom innerhalb der Abdeckhaube 6 gleichgerichtet, sodass sich die beiden Kräfte überlagern und eine hohe Trenn- und Saugwirkung erzielen.
Der Applikator 1 in der zweiten Ausführungsform nach den Fig. 4 - 7 entwickelt mehrere Wassertrennstrahlen, die sich gegenüberliegen. Damit werden die Wassertrennstrahlen fokussiert, wodurch das abzutrennende Gewebe allseitig untergraben, angehoben und im Fokus zerkleinert wird. Durch die Anordnung des Austrittsdüsen 11 nach der Fig. 7 wird ein zusätzlicher Drehimpuls auf das abzutrennende Gewebe ausgeübt, der das Trennen weiter erleichtert.
In den dritten und vierten Ausführungsformen nach den Fig. 8, 9, 12 und 13 ist der Wassertrennstrahl von der Saugöffnung 12 weggerichtet. Dabei wird der trennende Wasserstrahl von der runden Innenwand der Abdeckhaube 6", 6'" erfasst und umgelenkt, sodass der Wassertrennstrahl an der Innenwand entlang gleitet und zur Saugöffnung 12 zurückgeführt wird.

Nach dem erfolgten Debridement wird die Druckerzeugung für den Wasserstrahl abgeschaltet und die Bypassöffnung 13 vom Operateur geöffnet, sodass sich der Saugstrom entspannt und das an der Dichtkante 7 der Abdeckhaube 6, 6', 6", 6"' angesaugte Gewebe wieder freigibt. Danach wird der Applikator 1 ins nächste Arbeitsfeld umgesetzt und neu in Bearbeitung genommen.
Dieses Umsetzen des Applikators 1 innerhalb der Wunde oder des Geschwürs erübrigt sich bei der Ausführungsform nach der Fig. 12 und 13 weitestgehend, weil die glockenförmige Abdeckhaube 6"' so großvolumig ausgeführt ist, dass sie die Wunde oder das Geschwür in der Regel im Ganzen überdeckt. Die Bearbeitung Zug um Zug erfolgt hierbei durch die vertikale Anhebung und die horizontale Auslenkung der gebündelten Druckleitung 3 und Saugleitung 4 innerhalb der Arbeitskammer 8.
Der abgewandelte Applikator 1 in der dritten Ausführungsform nach den Fig. 10, 11 besitzt darüber hinaus gehende Funktionen und Vorteile. So ergibt der abgeschrägte Verlauf der distalen Dichtkante 7 eine ovale Arbeitsfläche am Körper des Patienten und damit einen größten möglichen Abstand zwischen der Austrittsdüse 11 und den Saugöffnungen 12. Mit dieser abgeschrägten Dichtkante 7 bekommt der Applikator 1 eine handlichere Arbeitsposition. Mit dem Verschließen der ersten Bypassöffnung 13 fließt ein Saugstrom über die zweite Bypassöffnung 20, der Arbeitskammer 8 und den Saugöffnungen 12 in der Saugleitung 4. Dieser Saugstrom bestimmt zunächst die Größe des Vakuums und sorgt auch während der Wasserstahltrennung für einen unterstützenden Mitreißeffekt für das abgetrennte Gewebe. Das auf Grund der Saugleistung der Vakuumpumpe einerseits und des Durchflusswiderstandes an der zweiten Bypassöffnung 20 andererseits entstehende Vakuum zieht das Körpergewebe des Patienten in abdichtender Weise gegen die Dichtkante 7. An besonderen Körperstellen des Patienten, die eine derartige Gewebeverschiebung nicht oder nicht ausreichend zulassen, wie es sich beispielsweise im Bereich von Gliedmaßen ergibt, saugt das Vakuum das flexible Dichtelement 16 in die Körperdellen und gewährleistet damit auch in diesen Fällen eine funktionsbedingte Abdichtung. Dieser Vakuumdruck wird vom Vakuumsensor 21 überwacht. Nachdem sich in der Arbeitskammer 8 ein vorbestimmter Vakuumdruck eingestellt hat, signalisiert der Vakuumsensor 21 einen Steuerbefehl zur Aktivierung der Flüssigkeitsförderpumpe. Der erzeugte Wasserstrahl verrichtet dann seine Arbeit in bereits beschriebener intelligenter Weise.
Nach dem erfolgten Debridement öffnet der Operateur die Bypassöffnung 13, sodass sich das Vakuum entspannt und sich das Dichtelement 16 von der Körperoberfläche löst. Der Vakuumsensor 21 erfasst diese Druckveränderung in der Arbeitskammer 8 und gibt ein Steuersignal an die Flüssigkeitsförderpumpe, um sie sofort abschalten zu lassen. Dieses Steuersignal leitet gleichzeitig eine kurze Umkehrung der Förderrichtung ein, um ein Nachlaufen der Flüssigkeitsförderpumpe und damit eine begrenzte Weiterförderung zu minimieren. Der Operateur hebt also unmittelbar nach dem Öffnen der ersten Bypassöffnung 13 den Appplikator 1 von der Körperoberfläche des Patienten ab, um es an einer nächsten Körperstelle wieder ansetzen zu können. Auf Grund der sensorgestützten An- und Abschaltung der Flüssigkeitsförderpumpe wird ein unkontrolliertes Austreten von mit Bakterien versetzter Flüssigkeit in das Umfeld unterbunden. Eine zusätzliche Sicherheit gegen das unerwünschte Austreten von verunreinigter Flüssigkeit tritt durch die besondere Funktion des Dichtelementes 16 ein. Auf Grund der natürlichen Bewegung des Operateurs löst sich beim Abheben des Applikators 1 zunächst der obere Teil der Dichtkante 7, in dessen Bereich sich die Austrittsdüse 11 befindet, während der untere Teil im Bereich der Saugöffnungen 12 noch an der Körperoberfläche verweilt. Dadurch kommt es zum Aufklappen der Fangklappe 19, die einen wider erwarten dennoch kurzzeitig vorhandenen Druckflüssigkeitsstrahl aufnimmt und in das Innere der Arbeitskammer 8 umlenkt. Ein Austreten des Druckflüssigkeitsstrahls aus dem Bereich der Arbeitskammer 8 in die Nähe des Operateurs wird dadurch verhindert. Nach dem Umsetzen des Applikators 1 wiederholt sich diese Funktion an einer anderen Körperposition.

### Liste der Bezugszeichen

- 1: Applikator
- 2: Handstück
- 3: Druckleitung
- 4: Saugleitung
- 5: Arbeitssonde
- 6: Abdeckhaube
- 7: Dichtkante
- 8: Arbeitskammer
- 9: nahe Seitenfläche
- 10: ferne Seitenfläche
- 11: Austrittsdüse
- 12: Saugöffnung
- 13: Bypassöffnung
- 14: flexibles Halte- und Dichtelement
- 15: Dichtelement
- 16: Dichtelement
- 17: Haltering
- 18: Dichtlippe
- 19: Fangklappe
- 20: zweite Bypassöffnung
- 21: Vakuumsensor
- 22: Druckfühlerleitung
- 23: Signalleitung

## Patentansprüche

1. Applikator für eine Wasserstrahltranneinrichtung, insbesondere zur Behandlung von Wunden und Geschwüren, bestehend aus einem Handgriff (2), einer Druckleitung (3) und einer Saugleitung (4), wobei die Druckleitung (3) mindestens ein Austrittsdüse (11) und die Saugleitung (4) mindestens eine Saugöffnung (12) besitzt und die Austrittsdüse (11) und die Saugöffnung (12) in einer Abdeckhaube (6, 6', 6", 6'") angeordnet sind und die Abdeckhaube (6, 6', 6", 6''') vorzugsweise aus einem transparenten Material besteht,
**dadurch gekennzeichnet, dass** die Abdeckhaube (6, 6', 6", 6"') mit Körperteilen des Patienten eine definiert vakuumverschlossene und von einem Vakuumsensor (21) überwachte Arbeitskammer (8) ausbildet.

2. Applikator nach Anspruch 1,
**dadurch gekennzeichnet, dass** die Austrittsdüse (11) der Druckleitung (3) auf die Saugöffnung (12) der Saugleitung (4) gerichtet ist.

3. Applikator nach Anspruch 1,
**dadurch gekennzeichnet, dass** die Austrittsdüse (11) radial vorzugsweise an der zum Handstück (2) nahen Seitenfläche (9) und die Saugöffnung (12) radial an der zum Handstück (2) fernen Seitenfläche (10) angeordnet sind.

4. Applikator nach Anspruch 1,
**dadurch gekennzeichnet, dass** die Abdeckhaube (6') an ihrem Umfang in einer oberen Ebene mindestens eine Austrittsdüse (11) und in einer unteren Ebene mindestens eine Saugöffnung (12) besitzt.

5. Applikator nach Anspruch 4,
**dadurch gekennzeichnet, dass** die Austrittsdüsen (11) so angeordnet sind, dass die austretenden Wasserstrahlen einen Focus ausbilden.

6. Applikator nach Anspruch 4,
**dadurch gekennzeichnet, dass** die Austrittsdüsen (11) so angeordnet sind, dass die austretenden Wasserstrahlen auf Abstand einander begegnen und einen mittigen Drall erzeugen.

7. Applikator nach Anspruch 2,
**dadurch gekennzeichnet, dass** die Abdeckhaube (6") vorzugsweise zylindrisch ausgebildet ist, die Druckleitung (3) und die Saugleitung (4) in der Länge umhüllt, und am distalen Ende offen ausgeführt ist, wobei die Druckleitung (3) und die Saugleitung (4) außermittig zur Abdeckhaube (6") in der Art angeordnet sind, dass sich die Austrittsdüse (11) der Druckleitung (3) und die Saugöffnung (12) der Saugleitung (4) im distalen Bereich mit einem größten radialen Abstand zueinander befinden.

8. Applikator nach Anspruch 1,
**dadurch gekenazeichnet, dass** die Austrittsdüse (11) der Druckleitung (3) radial und von der Saugöffnung (12) weg und gegen die Abdeckhaube (6", 6"') gerichtet ist.

9. Applikator nach Anspruch 8,
**dadurch gekennzeichnet, dass** die Abdeckhaube (6") vorzugsweise zylindrisch ausgebildet ist, die Druckleitung (3) und die Saugleitung (4) in der Länge umhüllt, und am distalen Ende offen ausgeführt ist, wobei die Druckleitung (3) und die Saugleitung (4) außermittig zur Abdeckhaube (6") in der Art angeordnet sind, dass sich die Saugleitung (4) achsfern und die Druckleitung (3) auf der achsnahen Seite der Saugleitung (4) befinden.

10. Applikator nach Anspruch 8,
**dadurch gekennzeichnet, dass** die Abdeckhaube (6"') glockenförmig ausgebildet ist und die Druckleitung (3) und die Saugleitung (4) in der Länge umhüllt, wobei die Druckleitung (3) und die Saugleitung (4) vorzugsweise parallel zusammen und in der Abdeckhaube gelenkig und vorstellbar gelagert sind.

11. Applikator nach Anspruch 10,
**dadurch gekennzeichnet, dass** mindestens ein Gelenk in der Abdeckhaube (6"') vorgesehen ist, das jeweils vorzugsweise als ein flexibles Halte- und Dichtelement (14) ausgebildet ist und welches bei Nichtbenutzung verschließbar ist.

12. Applikator nach Anspruch 1,
**dadurch gekennzeichnet, dass** die Abdeckhaube (6,6', 6", 6"') ein an die Körperteile anpassbares Dichtelement (15, 16) mit einer Dichtkante (7, 18) besitzt.

13. Applikator nach Anspruch 12,
**dadurch gekennzeichnet, dass** das Dichtelement (15, 16) im Strahlbereich der Austrittsdüse (11) eine Fangklappe (19) besitzt, die während der Abwendung des Applikators 1 in die Arbeitskammer (8) eingeklappt und die mit dem Abheben des Applikators von der Behandlungsstelle in Richtung der Körperoberfläche ausklappt.

14. Applikator nach Anspruch 1,
**dadurch gekennzeichnet, dass** in der Abdeckhaube (6, 6', 6", 6"') über die Saugleitung (4) ein vorzugsweise pulsierendes, das Gewebe stimulierendes Vakuum erzeugt wird.

15. Applikator nach Anspruch 1,
**dadurch gekennzeichnet, dass** an der Arbeitskammer (8) ein Vakuumsensor (21) angeschlossen ist, der über eine Signalleitung (23) in der Art mit der Flüssigkeitsföxderpumpe verbunden ist, dass die Flüssigkeitsförderpumpe nur innerhalb eines vorbestimmten Vakuumdruckbereiches förderfähig ist, der über den Vakuumsensor (21) ausgewertet wird.

16. Applikator nach Anspruch 15,
**dadurch gekennzeichnet, dass** der Vakuumsensor (21) in der Art mit der Flüssigkeitsförderpumpe verbunden ist, dass mit der Abschaltung der Flüssigkeitsförderpumpc die Förderrichtung der Flüssigkeitsförderpumpe kurzzeitig umgekehrt wird.

## Claims

1. An applicator for a water jet separating device, particularly for the treatment of wounds and abscesses, consisting of a hand piece (2), a pressure pipe (3), and a suction pipe (4), the pressure pipe (3) having at least one exit nozzle (11) and the suction pipe (4) having at least one suction port (12), and the exit nozzle (11) and the suction port (12) being arranged within a covering hood (6, 6', 6", 6"'), and the covering hood (6, 6', 6", 6"') preferably consisting of a transparent material,
**characterised in that** the covering hood (6, 6', 6", 6"') forms with parts of the body of the patient a working chamber (8) which is vacuum-sealed in a defined manner and is monitored by a vacuum sensor (21).

2. An applicator according to Claim 1,
**characterised in that** the exit nozzle (11) of the pressure pipe (3) is directed onto the suction port (12) of the suction pipe (4).

3. An applicator according to Claim 1,
**characterised in that** the exit nozzle (11) is radially arranged, preferably on the side face (9) proximal to the hand piece (2), and the suction port (12) is radially arranged on the side face (10) distal to the hand piece (2).

4. An applicator according to Claim 1,
**characterised in that** the covering hood (6') is provided on its circumference with at least one exit nozzle (11) on an upper level and with at least one suction port (12) on a lower level.

5. An applicator according to Claim 4,
**characterised in that** the exit nozzles (11) are arranged in such a manner that the emergent water jets form a focus.

6. An applicator according to Claim 4,
**characterised in that** the exit nozzles (11) are arranged in such a manner that the emergent water jets meet each other at a distance and generate a central swell.

7. An applicator according to Claim 2,
**characterised in that** the covering hood (6") is preferably cylindrical, envelops the lengths of the pressure pipe (3) and of the suction pipe (4), and is open at its distal end, the pressure pipe (3) and the suction pipe (4) being arranged eccentrically to the covering hood (6") in such a manner that the exit nozzle (11) of the pressure pipe (3) and the suction port (12) of the suction pipe (4) are located at a greatest radial distance to each other in the distal region.

8. An applicator according to Claim 1,
**characterised in that** the exit nozzle (11) of the pressure pipe (3) is directed radially and away from the suction port (12) and towards the covering hood (6", 6"').

9. An applicator according to Claim 8,
**characterised in that** the covering hood (6") is preferably cylindrical, envelops the lengths of the pressure pipe (3) and of the suction pipe (4), and is open at its distal end, the pressure pipe (3) and the suction pipe (4) being arranged eccentrically to the covering hood (6") in such a manner that the suction pipe (4) is located distal to the axis and the pressure pipe (3) is located on the side proximal to the axis of the suction pipe (4).

10. An applicator according to Claim 8,
**characterised in that** the covering hood (6"') is bell-shaped and envelops the lengths of the pressure pipe (3) and of the suction pipe (4), the pressure pipe (3) and the suction pipe (4) being preferably supported together in parallel so as to be articulatable and adjustable in the covering hood.

11. An applicator according to Claim 10,
**characterised in that** at least one joint is provided in the covering hood (6"'), which joint is preferably configured as a flexible retaining and sealing element (14) in each case and can be locked when not in use.

12. An applicator according to Claim 1,
**characterised in that** the covering hood (6, 6', 6", 6"') is provided with a sealing element (15, 16) having a sealing edge (7, 18) which can be adapted to the parts of the body.

13. An applicator according to Claim 12,
**characterised in that** the sealing element (15, 16) is provided with a catch flap (19) in the jet region of the exit nozzle (11), which catch flap folds into the working chamber (8) during use of the applicator (1) and folds out in the direction of the surface of the body when the applicator is lifted from the treatment site.

14. An applicator according to Claim 1,
**characterised in that** a preferably pulsating vacuum is generated in the covering hood (6, 6', 6", 6"') via the suction pipe (4), which vacuum stimulates the tissue.

15. An applicator according to Claim 1,
**characterised in that** a vacuum sensor (21) is connected to the working chamber (8), which vacuum sensor is connected to the liquid feed pump via a signal line (23) in such a manner that the liquid feed pump may pump only within a pre-determined vacuum pressure range which is evaluated by the vacuum sensor (21).

16. An applicator according to Claim 15,
**characterised in that** the vacuum sensor (21) is connected to the liquid feed pump in such a manner that the feed direction of the liquid feed pump is reversed during a short period of time when the liquid feed pump is switched off.

## Revendications

1. Applicateur pour un dispositif séparateur à jet d'eau, notamment pour le traitement de blessures et d'ulcères, composé d'une poignée (2), d'une conduite sous pression (3) et d'une conduite d'aspiration (4), la conduite sous pression (3) possédant au moins une buse de sortie (11), et la conduite d'aspiration (4) au moins une ouverture d'aspiration (12), et la buse de sortie (11) et l'ouverture d'aspiration (12) étant disposées dans un capot (6, 6', 6", 6"'), et le capot (6, 6', 6", 6"') se composant de préférence d'un matériau transparent,
**caractérisé en ce que** le capot (6, 6', 6", 6"') forme, avec des parties corporelles du patient, une chambre de travail (8) mise sous vide de façon définie et surveillée par un capteur de vide (21).

2. Applicateur selon la revendication 1,
**caractérisé en ce que** la buse de sortie (11) de la conduite sous pression (3) est dirigée vers l'ouverture d'aspiration (12) de la conduite d'aspiration (4).

3. Applicateur selon la revendication 1,
**caractérisé en ce que** la buse de sortie (11) est disposée de façon radiale de préférence sur la surface latérale (9) proche de la pièce à main (2), et **en ce que** l'ouverture d'aspiration (12) est disposée de façon radiale sur la surface latérale (10) éloignée de la pièce à main (2).

4. Applicateur selon la revendication 1,
**caractérisé en ce que** le capot (6') possède, sur son pourtour, dans une plan supérieur, au moins une buse de sortie (11) et, dans un plan inférieur, au moins une ouverture d'aspiration (12).

5. Applicateur selon la revendication 4,
**caractérisé en ce que** les buses de sortie (11) sont disposées de telle sorte que les jets d'eau sortants constituent un foyer.

6. Applicateur selon la revendication 4,
**caractérisé en ce que** les buses de sortie (11) sont disposées de telle sorte que les jets d'eau sortants se rencontrent à distance et produisent une rotation médiane.

7. Applicateur selon la revendication 2,
**caractérisé en ce que** le capot (6") est de préférence constitué de façon cylindrique, enveloppe la conduite sous pression (3) et la conduite d'aspiration (4) dans la longueur, et est réalisé de façon ouverte à l'extrémité distale, la conduite sous pression (3) et la conduite d'aspiration (4) étant disposées de façon décentrée par rapport au capot (6") de sorte que la buse de sortie (11) de la conduite sous pression (3) et l'ouverture d'aspiration (12) de la conduite d'aspiration (4) sont situées, dans la zone distale, avec un intervalle radial maximal entre elles.

8. Applicateur selon la revendication 1,
**caractérisé en ce que** la buse de sortie (11) de la conduite sous pression (3) est orientée dans le sens radial et de façon éloignée de l'ouverture d'aspiration (12) et vers le capot (6", 6"').

9. Applicateur selon la revendication 8,
**caractérisé en ce que** le capot (6") est de préférence constitué de façon cylindrique, enveloppe la conduite sous pression (3) et la conduite d'aspiration (4) dans la longueur, et est réalisé de façon ouverte à l'extrémité distale, la conduite sous pression (3) et la conduite d'aspiration (4) étant disposées de façon décentrée par rapport au capot (6") de sorte que la conduite d'aspiration (4) est placée éloignée de l'axe, et la conduite sous pression (3) est placée sur le côté de la conduite d'aspiration (4) proche de l'axe.

10. Applicateur selon la revendication 8,
**caractérisé en ce que** le capot (6"') est constitué en forme de cloche et enveloppe la conduite sous pression (3) et la conduite d'aspiration (4) dans la longueur, la conduite sous pression (3) et la conduite d'aspiration (4) étant supportées ensemble de préférence parallèlement et de façon réglable et articulée dans le capot.

11. Applicateur selon la revendication 10,
**caractérisé en ce qu'**au moins une articulation est prévue dans le capot (6"') et est constitué respectivement de préférence sous forme d'élément de retenue et d'étanchéité (14) flexible et peut être verrouillée en cas de non-utilisation.

12. Applicateur selon la revendication 1,
**caractérisé en ce que** le capot (6, 6', 6", 6"') possède un élément d'étanchéité (15, 16), adaptable aux parties corporelles, avec une arête d'étanchéité (7, 18).

13. Applicateur selon la revendication 12,
**caractérisé en ce que** l'élément d'étanchéité (15, 16) possède dans la zone du jet de la buse de sortie (11) un clapet collecteur (19) qui, pendant l'utilisation de l'applicateur 1, se replie dans la chambre de travail (8) et qui se déploie avec le soulèvement de l'applicateur depuis l'emplacement de traitement en direction de la surface corporelle.

14. Applicateur selon la revendication 1,
**caractérisé en ce que**, dans le capot (6, 6', 6", 6"'), un vide, de préférence pulsatoire, stimulant le tissu, est produit par le biais de la conduite d'aspiration (4).

15. Applicateur selon la revendication 1,
**caractérisé en ce qu'**un capteur de vide (21) est raccordé sur la chambre de travail (8) et est raccordé, par le biais d'une ligne de signalisation (23), à la pompe d'alimentation en liquide de sorte que la pompe d'alimentation en liquide ne peut fonctionner qu'à l'intérieur d'une plage de dépression prédéfinie qui est analysée par le biais du capteur de vide (21).

16. Applicateur selon la revendication 15,
**caractérisé en ce que** le capteur de vide (21) est raccordé à la pompe d'alimentation en liquide de sorte que, lors de l'arrêt de la pompe d'alimentation en liquide, le sens de circulation de la pompe d'alimentation en liquide est brièvement inversé.
